# EUROPEAN PATENT APPLICATION

(11) **EP 4 712 097 A1**
(43) Date of publication of application: **18.03.2026**
(21) Application number: 24306507.5
(22) Date of filing: 13.09.2024
(51) Int. Cl.: G16H 50/20

(54) **ELECTROCARDIOGRAM ANALYSIS SYSTEM**

(71) Applicant: Koninklijke Philips N.V., 5656 AG Eindhoven (NL)
(72) Inventor: PEZZOTTI, Nicola, 5656 Eindhoven (NL); SHRESHTHA, Kumar, 5656 Eindhoven (NL); CHOFFIN, Benoît, 5656 Eindhoven (NL); KHELDOUNI, Amine, 5656 Eindhoven (NL); REBENA, Nicolas, 5656 Eindhoven (NL)
(74) Representative: Philips Intellectual Property & Standards

(57) **Abstract**

The present disclosure provides concepts for analyzing electrocardiogram (ECG) data of a subject. Specifically, a traditional prediction unit is configured to process the ECG data with a rules-based ECG interpretation model to generate first diagnostic labels indicating presence or absence of cardiac conditions. A machine learning prediction unit processes the ECG data with a machine learning-based ECG interpretation model to generate second diagnostic labels indicating presence or absence of cardiac conditions. A mapping unit determines a feature mapping describing first and second diagnostic labels associated with similar cardiac conditions. A combination unit analyzes the first diagnostic labels based on the feature mapping and the second diagnostic labels to generate output diagnostic labels indicating presence or absence of cardiac conditions of the subject. The provided concepts therefore leverage the benefits of both the rules-based ECG interpretation model and the machine learning-based ECG interpretation model to provide output diagnostic labels. Specifically, the granularity of the output of the rules-based ECG interpretation model may be maintained whilst ensuring accuracy via the output of the machine learning-based ECG interpretation model.

## Description

### FIELD OF INVENTION

The present invention relates to systems and methods for analyzing electrocardiogram (ECG) data, and more particularly to combining traditional rule-based models with machine learning models to improve ECG interpretation and diagnosis.

### BACKGROUND

Electrocardiograms (ECGs) are widely used as a primary diagnostic tool for cardiovascular diseases due to their relative ease and speed of acquisition, cheap measurement devices, and ability to provide valuable insights into a subject's cardiac status. However, interpreting ECG data requires significant expertise and a deep understanding of cardiac electrophysiology. This complexity creates a challenge in leveraging ECGs to their full potential, especially in settings where expert cardiologists may not be readily available.

Traditional ECG interpretation models usually rely on a set of rules that apply predefined criteria to identify specific patterns and anomalies. While these models can provide automated diagnoses, they often lack the sensitivity and specificity required for reliable clinical decision-making. As a result, human interpretation by trained professionals remains necessary, which can be time-consuming and subject to errors.

Recent advancements in artificial intelligence (AI) and machine learning have shown promise in improving ECG analysis. AI and machine learning based models can potentially offer more accurate and consistent interpretations by learning from large datasets of annotated ECGs. However, these models face limitations in terms of the number of conditions they can reliably diagnose, as training on a comprehensive set of cardiac abnormalities presents significant challenges in data acquisition and annotation.

The current landscape of ECG interpretation thus presents a dilemma: traditional models offer broad coverage of diagnostic labels but with limited accuracy, while AI models provide improved accuracy but for a reduced set of conditions. This situation highlights the need for innovative approaches that can leverage the strengths of both traditional and AI-based methods to enhance ECG interpretation and diagnosis.

### SUMMARY OF INVENTION

The invention is defined by the claims.

According to an aspect of the present disclosure, a system for analyzing electrocardiogram (ECG) data of a subject is provided. The system includes a traditional prediction unit configured to process, with a rules-based ECG interpretation model, the ECG data to generate first diagnostic labels indicating presence or absence of cardiac conditions of the subject. The system also includes a machine learning prediction unit configured to process, with a machine learning-based ECG interpretation model, the ECG data to generate second diagnostic labels indicating presence or absence of cardiac conditions of the subject. Additionally, the system comprises a mapping unit configured to determine a feature mapping describing first diagnostic labels and second diagnostic labels associated with similar cardiac conditions. Furthermore, the system includes a combination unit configured to analyze the first diagnostic labels based on the feature mapping and the second diagnostic labels, to generate output diagnostic labels indicating presence or absence of cardiac conditions of the subject.

Proposed concepts thus aim to provide schemes, solutions, concepts, designs, methods, and systems pertaining to electrocardiogram (ECG) analysis. In particular, embodiments aim to combine traditional rule-based models with machine learning models. This hybrid system leverages the strengths of both approaches to improve ECG interpretation and diagnosis.

The main concept of the invention is the integration of a rules-based ECG interpretation model with a machine learning-based ECG interpretation model. The rules-based model generates a comprehensive set of first diagnostic labels, while the machine learning model provides more accurate predictions for a subset of cardiac conditions relating to a set of second diagnostic labels. A key innovation is the use of a feature mapping that describes the relationship between the diagnostic labels from both models, allowing for a sophisticated combination of their outputs.

In other words, the invention leverages the idea that traditional models can provide a wide range of diagnostic labels, including those detailing cardiac sub-conditions, while machine learning models excel at accurately identifying general cardiac conditions. By combining these approaches, the system can maintain the granularity of traditional diagnostics while benefiting from the improved accuracy of machine learning predictions.

Thus, the proposed invention combines the output of rules-based ECG interpretation models with machine-learning ECG interpretation models. It has been realized that machine-learning ECG interpretation models typically provide far fewer diagnostic labels than rules-based ECG interpretation models (e.g., due to training set and computational limitations). Nevertheless, machine-learning ECG interpretation models are generally much more accurate, with a lower rate of false positive. Therefore, it is proposed to analyze the output from the rules-based ECG interpretation model (e.g., the first diagnostic labels) with the output from the machine-learning ECG interpretation models (e.g., the second diagnostic labels), and a feature mapping describing the relationship therebetween. Accordingly, the output diagnostic labels may have the same level of granularity as the output of the rules-based ECG interpretation model (i.e., still relate to a same/similar number of diagnostic labels), whilst benefiting from the improved accuracy of the machine-learning based ECG interpretation model.

In some embodiments, the combination unit may be configured, for each first diagnostic label, to determine a validity of the first diagnostic label by comparison to associated second diagnostic labels, and provide output diagnostic labels based on the valid first diagnostic labels.

One of the primary advantages of this embodiment is its ability to reduce false positives and improve the specificity of ECG diagnoses. The system uses the machine learning model's predictions to validate or invalidate the diagnoses made by the traditional model, effectively filtering out potential overdiagnoses. This approach significantly enhances the reliability of ECG interpretations, potentially reducing the need for unnecessary follow-up tests or treatments.

More specifically, the combination unit may be configured, for each first diagnostic label, to determine whether one of the associated second diagnostic labels contradicts the respective first diagnostic label, and identify the first diagnostic label as invalid. This feature helps in reducing false positives by cross-validating the traditional model's predictions with the machine learning model's outputs.

In particular, for each first diagnostic label indicating the presence of a cardiac condition, the combination unit may determine whether one of the associated second diagnostic labels indicates the absence of the cardiac condition, and identify the first diagnostic label as invalid. This feature further enhances the specificity of the diagnosis by filtering out potential overdiagnoses from the traditional model.

In some embodiments, the traditional prediction unit may be configured to generate a first number of first diagnostic labels, while the machine learning prediction unit may be configured to generate a second number of second diagnostic labels, where the first number is greater than the second number. This feature allows for more granular diagnoses from the traditional model while benefiting from the higher accuracy of the machine learning model on broader categories.

In specific cases, the second diagnostic labels may indicate the presence or absence of general cardiac conditions, and the first diagnostic labels may indicate the presence or absence of general cardiac conditions and cardiac sub-conditions. This feature provides a more detailed diagnosis while still maintaining high accuracy in general conditions.

The machine learning-based ECG interpretation model may be a deep-learning model, which can potentially capture more complex patterns in the ECG data.

The rules-based ECG interpretation model may comprise a set of parameters based on which the ECG data is assessed to identify the presence or absence of cardiac conditions in the ECG data. These parameters may be adapted to increase the identification of the presence of cardiac conditions in the ECG data, potentially improving the sensitivity of the traditional model.

In some embodiments, the combination unit may be configured to generate the same number of output diagnostic labels as the number of first diagnostic labels, ensuring that the final output maintains the granularity of the traditional model.

Thus, this embodiment provides the key advantage of providing more comprehensive and accurate ECG analysis without sacrificing the familiar output format that clinicians are accustomed to from traditional models. By generating the same number and type of diagnostic labels as traditional models, but with improved accuracy, the invention offers a solution that can be more readily adopted in clinical settings. This balance between innovation and familiarity could lead to faster integration of advanced ECG analysis techniques in healthcare practices, ultimately improving patient care and outcomes in cardiovascular diagnostics.

The system may further comprise a display configured to present the output diagnostic labels, facilitating easy interpretation of the results by healthcare professionals.

According to another aspect of the present disclosure, a method for analyzing electrocardiogram (ECG) data of a subject is provided. The method includes processing, with a rules-based ECG interpretation model, the ECG data to generate first diagnostic labels indicating presence or absence of cardiac conditions of the subject. The method also includes processing, with a machine learning-based ECG interpretation model, the ECG data to generate second diagnostic labels indicating presence or absence of cardiac conditions of the subject. Additionally, the method comprises determining a feature mapping describing first diagnostic labels and second diagnostic labels associated with similar cardiac conditions. Furthermore, the method includes analyzing the first diagnostic labels based on the feature mapping and the second diagnostic labels, to generate output diagnostic labels indicating presence or absence of cardiac conditions of the subject.

According to another aspect of the present disclosure, a computer program comprising computer program code means adapted, when said computer program is run on a computer, to implement the method described above is provided. This aspect allows for the efficient implementation of the analysis method on various computing devices.

These and other aspects of the invention will be apparent from and elucidated with reference to the embodiment(s) described hereinafter.

### BRIEF DESCRIPTION OF FIGURES

For a better understanding of the invention, and to show more clearly how it may be carried into effect, reference will now be made, by way of example only, to the accompanying drawings, in which:
FIG. 1 illustrates a block diagram of a system for analyzing electrocardiogram (ECG) data according to aspects of the present disclosure;
FIG. 2 illustrates a flowchart for a method of analyzing ECG data according to an embodiment; and
FIG. 3 is a simplified block diagram of a computer within which one or more parts of an embodiment may be employed.

### DETAILED DESCRIPTION

The invention will be described with reference to the Figures.

It should be understood that the Figures are merely schematic and are not drawn to scale. It should also be understood that the same reference numerals are used throughout the Figures to indicate the same or similar parts.

It should also be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to provide an advantage.

The present disclosure provides concepts for analyzing electrocardiogram (ECG) data of a subject. Specifically, a traditional prediction unit is configured to process the ECG data with a rules-based ECG interpretation model to generate first diagnostic labels indicating presence or absence of cardiac conditions. A machine learning prediction unit processes the ECG data with a machine learning-based ECG interpretation model to generate second diagnostic labels indicating presence or absence of cardiac conditions. A mapping unit determines a feature mapping describing first and second diagnostic labels associated with similar cardiac conditions. A combination unit analyzes the first diagnostic labels based on the feature mapping and the second diagnostic labels to generate output diagnostic labels indicating presence or absence of cardiac conditions of the subject. The provided concepts therefore leverage the benefits of both the rules-based ECG interpretation model and the machine learning-based ECG interpretation model to provide output diagnostic labels. Specifically, the granularity of the output of the rules-based ECG interpretation model may be maintained whilst ensuring accuracy via the output of the machine learning-based ECG interpretation model.

In other words, the present disclosure provides systems and methods for analyzing ECG data, that may leverage the strengths of both traditional rule-based models and machine learning models to enhance the interpretation and diagnosis of ECG data. Traditional rule-based models, which typically generate a comprehensive set of diagnostic labels, may be combined with machine learning models, which generally provide more accurate predictions for a subset of cardiac conditions. A feature mapping may be used to describe the relationship between the diagnostic labels from both models, allowing for a sophisticated combination of their outputs. This approach may maintain the granularity of traditional diagnostics while benefiting from the improved accuracy of machine learning predictions.

It has been realized that, whilst traditional models (either rule-based or signal-processing based models) enable automated analysis of ECGs, they are often not very reliable. Indeed, traditional models may provide for predictions related to hundreds of diagnostic labels related to a large array of cardiac conditions and sub-categories of those conditions. However, despite best efforts to improve such algorithms human interpretation is still required to ensure accuracy and reliability of the resulting diagnosis.

Artificial intelligence and machine learning based models provide one solution to the problem of accuracy. Such models may produce highly accurate diagnostic labels. However, such models need to be trained on large, labelled data sets. Thus, for such models to be trained, data sets of unrealistic size and quality are required to be able to accurately predict a similar number of diagnostic labels as traditional models. Equally, it is unacceptable to many users to reduce the number of diagnostic labels produced.

Accordingly, it is proposed to combine the outputs of the both of these types of models in order to provide an accurate, granular prediction. Specifically, a mapping representing relationships between the diagnostic labels produced by the different models is produced. Using this mapping, and the second diagnostic labels produced by the machine learning based ECG interpretation model, the first diagnostic labels produced by the rules-based ECG interpretation model may be analyzed. Accordingly, output diagnostic labels are generated, which may provide an accurate assessment at the same level of granularity as presently used models.

The proposed approach may reduce false positives and improve the specificity of ECG diagnoses by using the machine learning model's predictions to validate or invalidate the diagnoses made by the traditional model. This approach may significantly enhance the reliability of ECG interpretations, potentially reducing the need for unnecessary follow-up tests or treatments. Furthermore, proposed concepts may also provide more comprehensive and accurate ECG analysis without sacrificing the familiar output format (and output granularity) that clinicians are accustomed to. By generating the same number and type of diagnostic labels as traditional models, but with improved accuracy, the system and method may offer a solution that can be more readily adopted in clinical settings. This balance between innovation and familiarity could lead to faster integration of advanced ECG analysis techniques in healthcare practices, ultimately improving patient care and outcomes in cardiovascular diagnostics.

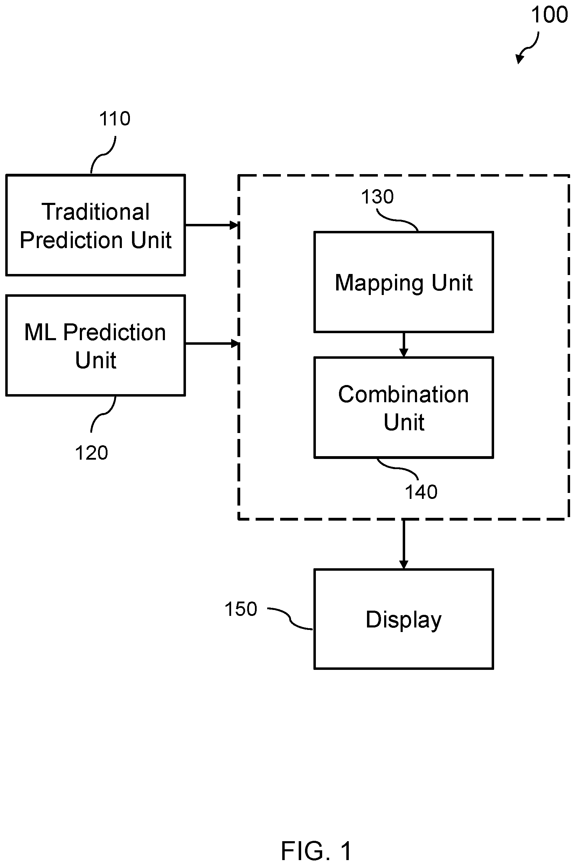

Referring now to FIG. 1, there is illustrated a block diagram of a system for analyzing ECG data according to an embodiment of the invention. The system 100 includes a traditional prediction unit 110, a machine learning prediction unit 120, a mapping unit 130, and a combination unit 140. The system may further comprise a display 150.

The traditional prediction unit 110 is configured to process the ECG data with a rules-based ECG interpretation model. This rules-based model may be based on a set of handcrafted domain-specific rules to diagnose an ECG. The traditional prediction unit 110 generates first diagnostic labels indicating the presence or absence of cardiac conditions of the subject. These first diagnostic labels may cover a large number of conditions, providing a comprehensive analysis of the ECG data.

For sake of clarity, each diagnostic labels relates to a certain potential cardiac condition. The diagnostic label may either indicate that the cardiac condition is present (based on the ECG data), or that the cardiac condition is not present. In this way, the plurality of diagnostic labels are essentially a list of potential heart cardiac conditions that are either flagged "true" or "false" to indicate whether or not the subject is believed to have a certain cardiac condition.

The rules-based ECG interpretation model used by the traditional prediction unit 110 may comprise a set of parameters based on which the ECG data is assessed to identify the presence or absence of cardiac conditions. These parameters may be handcrafted based on domain-specific knowledge and may cover a wide range of cardiac conditions and sub-conditions.

In some aspects, the set of parameters of the rules-based ECG interpretation model may be adapted to increase the identification of the presence of cardiac conditions in the ECG data. This may involve adjusting the parameters to be more sensitive, potentially at the expense of generating more false positives. However, these false positives may be filtered out by the combination unit 140, leveraging the more accurate predictions of the machine learning prediction unit 120. This will be described in more detail below.

The machine learning prediction unit 120 is configured to process the same ECG data with a machine learning-based ECG interpretation model. This model may be based on artificial intelligence techniques, such as deep learning, and may be trained to diagnose a more limited number of labels compared to the traditional prediction unit 110. The machine learning prediction unit 120 generates second diagnostic labels indicating the presence or absence of cardiac conditions of the subject. These second diagnostic labels may provide a more accurate diagnosis for the conditions they cover, compared to the first diagnostic labels.

The machine learning-based ECG interpretation model used by the machine learning prediction unit 120 may be a deep-learning model in some embodiments. Deep learning models are a type of artificial intelligence model that can learn complex patterns in data, making them well-suited for tasks such as ECG interpretation.

In some aspects, the traditional prediction unit 110 may generate a first number of first diagnostic labels, and the machine learning prediction unit 120 may generate a second number of second diagnostic labels, where the first number is greater than the second number. This reflects the more comprehensive nature of the traditional prediction unit 110, which may cover a larger number of conditions compared to the machine learning prediction unit 120.

Generally, the second diagnostic labels generated by the machine learning prediction unit 120 may indicate the presence or absence of general cardiac conditions (e.g., high level diagnoses). On the other hand, the first diagnostic labels generated by the traditional prediction unit 110 may indicate the presence or absence of both general cardiac conditions and cardiac sub-conditions (e.g., both high level diagnoses, as well as related conditions and other factors).

The mapping unit 130 may be configured to determine a feature mapping that describes the relationship between the first diagnostic labels and the second diagnostic labels. This feature mapping may associate similar cardiac conditions indicated by the first and second diagnostic labels. In some cases, the mapping unit 130 may map multiple first diagnostic labels to a single second diagnostic label, reflecting the more granular nature of the first diagnostic labels.

For example, one second diagnostic label may indicate the presence or absence of right ventricular hypertrophy, whilst many first diagnostic labels mapped to the second diagnostic labels may indicate the presence or absence of various features in the ECG related to right ventricular hypertrophy. The mapped first diagnostic labels may generally provide further sub-specifications of right ventricular hypertrophy, for example.

To be clear, each one of the first diagnostic labels may be associated with one or more of the second diagnostic labels. Some of the first diagnostic labels may not be associated with any second diagnostic labels. Furthermore, each one of the second diagnostic labels may be associated with one or more of the first diagnostic labels. Some of the second diagnostic labels may not be associated with any first diagnostic labels. In addition, the feature mapping may describe the type of association between the first diagnostic label and second diagnostic label (e.g., sub-categories, inter-related, etc.). The feature mapping may also indicate a strength of association between the diagnostic labels (e.g., causal, likely/incidental, etc.).

The combination unit 140 is configured to analyze the first diagnostic labels based on the feature mapping and the second diagnostic labels. The combination unit 140 then generates output diagnostic labels indicating the presence or absence of cardiac conditions of the subject. These output diagnostic labels may provide a more accurate and comprehensive diagnosis of the ECG data, leveraging the strengths of both the traditional prediction unit 110 and the machine learning prediction unit 120.

The combination unit 140 may be configured to determine the validity of each first diagnostic label by comparing it to the associated (according to the feature mapping) second diagnostic labels. The combination unit 140 may then provide output diagnostic labels based on the valid first diagnostic labels.

The analysis by the combination unit 140 may involve comparing each first diagnostic label with the associated second diagnostic labels to determine its validity. If a first diagnostic label is contradicted by the associated second diagnostic labels, it may be identified as invalid (as the machine learning-based model is more likely to be accurate than the rules-based model). If invalid, the first diagnostic label may be discarded.

More specifically, the combination unit 140 may determine whether one of the associated second diagnostic labels contradicts the respective first diagnostic label. For instance, if a first diagnostic label indicates the presence of a cardiac condition, but the associated second diagnostic label indicates the absence of the same condition, the combination unit 140 may identify the first diagnostic label as invalid.

In some cases, for each first diagnostic label indicating the presence of a cardiac condition, the combination unit 140 may determine whether one of the associated second diagnostic labels indicates the absence of the cardiac condition. If the second diagnostic label indicates the absence of the condition, the combination unit 140 may identify the first diagnostic label as invalid. This process may help to reduce false positives and improve the specificity of the ECG diagnoses.

In some aspects, the combination unit 140 may be configured to generate a same number of output diagnostic labels as the number of first diagnostic labels generated by the traditional prediction unit 110. Accordingly, from the viewpoint of the user, the granularity of the diagnostic labels is preserved, providing a detailed and comprehensive analysis of the ECG data. By maintaining the same number and type of diagnostic labels as traditional models, the system 100 may offer a solution that can be more readily adopted in clinical settings, as existing practice may not need to be adapted. This balance between innovation and familiarity could lead to faster integration of advanced ECG analysis techniques in healthcare practices, ultimately improving patient care and outcomes in cardiovascular diagnostics.

Overall, the system 100 may preserve the characteristics and sensitivity of traditional models when diagnosing cardiac abnormalities based on ECG interpretation. This is achieved by leveraging the strengths of the traditional prediction unit 110, such as its ability to generate a comprehensive set of diagnostic labels, while also benefiting from the improved accuracy of the machine learning prediction unit 120. By combining these two approaches, the system 100 may provide a more accurate and detailed diagnosis of the ECG data, potentially reducing the need for unnecessary follow-up tests or treatments.

Finally, the system 100 may further include a display 150 configured to present the output diagnostic labels. The display 150 may provide a visual representation of the output diagnostic labels, allowing clinicians to easily interpret the results of the ECG analysis. In some cases, the display 150 may present the output diagnostic labels in a format that is familiar to clinicians, further facilitating the adoption of the system 100 in clinical settings.

It has been shown that the above defined system can improve the specificity of cardiac condition detection in ECG data. In one example, the specificity of atrial fibrillation detection was shown to increase from 95.2% (using just the traditional model) to 98.9% with the combined approach. This demonstrates an improvement in specificity that may be attributed to the integration of the machine learning prediction unit's 120 predictions into the analysis of the traditional prediction unit's 110 diagnostic labels. The machine learning prediction unit 120, which may be trained on a more limited number of labels, may provide more accurate predictions for the conditions it covers. These accurate predictions may be used by the combination unit 140 to validate or invalidate the diagnoses made by the traditional prediction unit 110, thereby reducing false positives and improving the specificity of the ECG diagnoses.

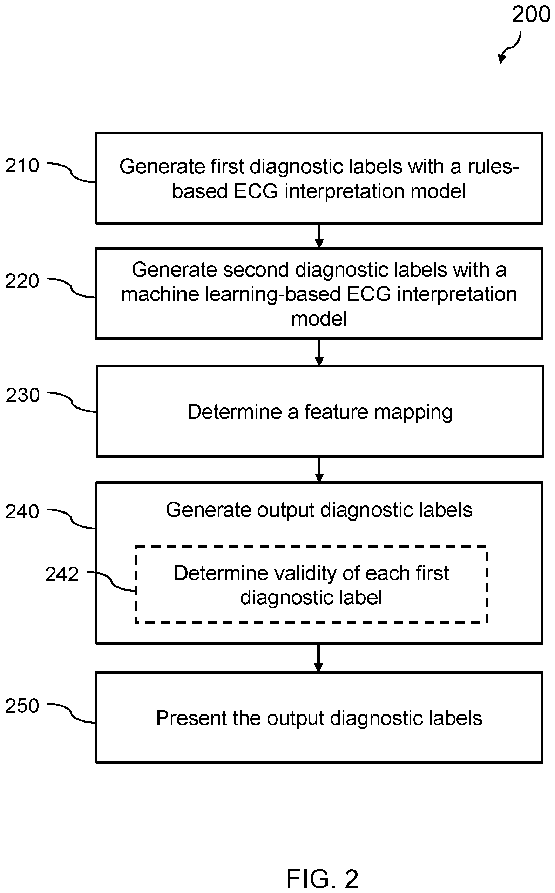

Referring to FIG. 2, a method 200 for analyzing ECG data of a subject is illustrated.

In step 210, ECG data is processed with a rules-based ECG interpretation model to generate first diagnostic labels indicating the presence or absence of cardiac conditions of the subject. This step may be performed by a traditional prediction unit, such as the traditional prediction unit 110 in FIG. 1. The rules-based ECG interpretation model may be based on a set of handcrafted domain-specific rules to diagnose an ECG. In some cases, the traditional prediction unit may generate a large number of first diagnostic labels, providing a comprehensive analysis of the ECG data.

In some cases, there may be a further step of adjusting parameters of the rules-based ECG interpretation model to increase sensitivity of the model. This may result in a higher number of false positives. However, as described, this false positive rate is offset by the machine learning-based ECG interpretation model that is used to effectively filter the results from the rules-based ECG interpretation model.

Next, in step 220, the same ECG data is processed with a machine learning-based ECG interpretation model to generate second diagnostic labels indicating the presence or absence of cardiac conditions of the subject. This step may be performed by a machine learning prediction unit, such as the machine learning prediction unit 120 in FIG. 1. The machine learning-based ECG interpretation model may be based on artificial intelligence techniques, such as deep learning, and may be trained to diagnose a more limited number of labels compared to the traditional prediction unit. The machine learning prediction unit may generate second diagnostic labels that provide a more accurate diagnosis for the conditions they cover, compared to the first diagnostic labels.

In step 230, a feature mapping is determined that describes the relationship between the first diagnostic labels and the second diagnostic labels. This step may be performed by a mapping unit, such as the mapping unit 130 in FIG. 1. The feature mapping may associate similar cardiac conditions indicated by the first and second diagnostic labels. In some cases, the feature mapping may map multiple first diagnostic labels to a single second diagnostic label, reflecting the more granular nature of the first diagnostic labels. The feature mapping may also describe a degree to which the first and second diagnostic labels are associated and/or how they are associated.

In step 240, the first diagnostic labels are analyzed based on the feature mapping and the second diagnostic labels to generate output diagnostic labels indicating the presence or absence of cardiac conditions of the subject. This step may be performed by a combination unit, such as the combination unit 140 in FIG. 1.

More specifically, in step 242, the first diagnostic labels may be compared with the associated second diagnostic labels (according to the feature mapping) to determine its validity. If a first diagnostic label is contradicted by the associated second diagnostic labels, it may be identified as invalid and discarded. The combination unit may then generate output diagnostic labels that provide a more accurate and comprehensive diagnosis of the ECG data, leveraging the strengths of both the traditional prediction unit and the machine learning prediction unit.

In step 250, the output diagnostic labels are presented to a user. For example, they may be output visually on a screen. Alternatively, the output diagnostic labels may be stored for future reference and review.

Of course, the method 200 may be implemented in a system for analyzing ECG data, such as the system 100 in FIG. 1. The method 200 leverages the strengths of both traditional rule-based models and machine learning models to enhance the interpretation and diagnosis of ECG data. By combining these two approaches, the method 200 may provide a more accurate and detailed diagnosis of the ECG data, potentially reducing the need for unnecessary follow-up tests or treatments. The method 200 may also provide more comprehensive and accurate ECG analysis without sacrificing the familiar output format that clinicians are accustomed to. By generating the same number and type of diagnostic labels as traditional models, but with improved accuracy, the method 200 may offer a solution that can be more readily adopted in clinical settings. This balance between innovation and familiarity could lead to faster integration of advanced ECG analysis techniques in healthcare practices, ultimately improving patient care and outcomes in cardiovascular diagnostics.

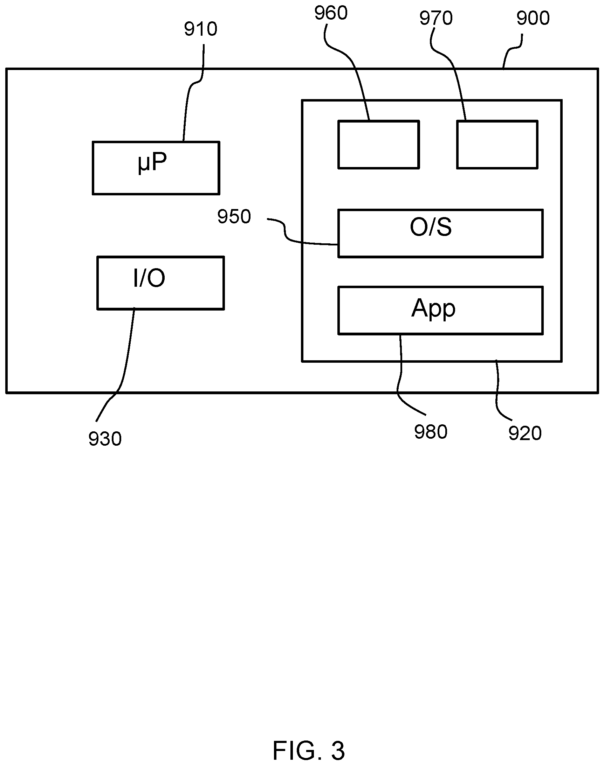

Fig. 3 illustrates an example of a computer 900 within which one or more parts of an embodiment may be employed. Various operations discussed above may utilize the capabilities of the computer. For example, one or more parts of a proposed embodiment may be incorporated in any element, module, application, and/or component discussed herein. In this regard, it is to be understood that system functional blocks can run on a single computer or may be distributed over several computers and locations (e.g. connected via internet), such as a cloud-based computing infrastructure.

The computer 900 includes, but is not limited to, PCs, workstations, laptops, PDAs, palm devices, servers, storages, and the like. Generally, in terms of hardware architecture, the computer 900 may include one or more processors 910, memory 920 and one or more I/O devices 930 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 910 is a hardware device for executing software that can be stored in the memory 920. The processor 910 can be virtually any custom made or commercially available processor, a central processing unit (CPU), a digital signal processor (DSP), or an auxiliary processor among several processors associated with the computer 900, and the processor 910 may be a semiconductor based microprocessor (in the form of a microchip) or a microprocessor.

The memory 920 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 920 may incorporate electronic, magnetic, optical, and/or other types of storage media. Note that the memory 920 can have a distributed architecture, where various components are situated remote from one another, but can be accessed by the processor 910.

The software in the memory 920 may include one or more separate programs, each of which comprises an ordered listing of executable instructions for implementing logical functions. The software in the memory 920 includes a suitable operating system (O/S) 950, compiler 960, source code 970, and one or more applications 980 in accordance with exemplary embodiments. As illustrated, the application 980 comprises numerous functional components for implementing the features and operations of the exemplary embodiments. The application 980 of the computer 900 may represent various applications, computational units, logic, functional units, processes, operations, virtual entities, and/or modules in accordance with exemplary embodiments, but the application 980 is not meant to be a limitation.

The operating system 950 controls the execution of other computer programs, and provides scheduling, input-output control, file and data management, memory management, and communication control and related services. It is contemplated by the inventors that the application 980 for implementing exemplary embodiments may be applicable on all commercially available operating systems.

Application 980 may be a source program, executable program (object code), script, or any other entity comprising a set of instructions to be performed. When a source program, then the program is usually translated via a compiler (such as the compiler 960), assembler, interpreter, or the like, which may or may not be included within the memory 920, so as to operate properly in connection with the O/S 950. Furthermore, the application 980 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 930 may include input devices such as, for example but not limited to, a mouse, keyboard, scanner, microphone, camera, etc. Furthermore, the I/O devices 930 may also include output devices, for example but not limited to a printer, display, etc. Finally, the I/O devices 930 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 900 is a PC, workstation, intelligent device or the like, the software in the memory 920 may further include a basic input output system (BIOS) (omitted for simplicity). The BIOS is a set of essential software routines that initialize and test hardware at start-up, start the O/S 950, and support the transfer of data among the hardware devices. The BIOS is stored in some type of read-only-memory, such as ROM, PROM, EPROM, EEPROM or the like, so that the BIOS can be executed when the computer 900 is activated.

When the computer 900 is in operation, the processor 910 is configured to execute software stored within the memory 920, to communicate data to and from the memory 920, and to generally control operations of the computer 900 pursuant to the software. The application 980 and the O/S 950 are read, in whole or in part, by the processor 910, perhaps buffered within the processor 910, and then executed.

When the application 980 is implemented in software it should be noted that the application 980 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 980 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed method(s), device(s) and/or system(s) may be implemented in hardware or software, or a mixture of both (for example, as firmware running on a hardware device). To the extent that an embodiment is implemented partly or wholly in software, the functional steps illustrated in the process flow diagrams may be performed by suitably programmed physical computing devices, such as one or more central processing units (CPUs) or graphics processing units (GPUs). Each process - and its individual component steps as illustrated in the flow diagrams - may be performed by the same or different computing devices. According to embodiments, a computer-readable storage medium stores a computer program comprising computer program code configured to cause one or more physical computing devices to carry out a control method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, some of the blocks shown in the block diagrams may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flow diagrams and block diagrams in the Figures illustrate the architecture, functionality, and operation of possible implementations of systems, methods, and computer program products according to various embodiments of the present invention. In this regard, each block in the flow diagrams or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flow diagrams and combinations of blocks in the block diagrams and/or flow diagrams, can be implemented by special purpose hardware-based systems that perform the specified functions or acts or carry out combinations of special purpose hardware and computer instructions.

## Claims

1. A system (100) for analyzing electrocardiogram, ECG, data of a subject, the system comprising:
a traditional prediction unit (110) configured to process, with a rules-based ECG interpretation model, the ECG data to generate first diagnostic labels indicating presence or absence of cardiac conditions of the subject;
a machine learning prediction unit (120) configured to process, with a machine learning-based ECG interpretation model, the ECG data to generate second diagnostic labels indicating presence or absence of cardiac conditions of the subject;
a mapping unit (130) configured to determine a feature mapping describing first diagnostic labels and second diagnostic labels associated with similar cardiac conditions; and
a combination unit (140) configured to analyze the first diagnostic labels based on the feature mapping and the second diagnostic labels, to generate output diagnostic labels indicating presence or absence of cardiac conditions of the subject.

2. The system of claim 1, wherein the combination unit (140) is configured, for each first diagnostic label, to determine a validity of the first diagnostic label by comparison to associated second diagnostic labels; and provide output diagnostic labels based on the valid first diagnostic labels.

3. The system of claim 2, wherein the combination unit (140) is configured, for each first diagnostic label, to:
determine whether one of the associated second diagnostic labels contradicts the respective first diagnostic label; and
identify the first diagnostic label as invalid.

4. The system of claim 3, wherein the combination unit (140) is configured, for each first diagnostic label indicating the presence of a cardiac condition, to:
determine whether one of the associated second diagnostic labels indicates the absence of the cardiac condition; and
identify the first diagnostic label as invalid.

5. The system of claim 1, wherein the traditional prediction unit (110) is configured to generate, with the rules-based ECG interpretation model, a first number of first diagnostic labels, the machine learning prediction unit (120) is configured to generate, with the ECG interpretation model, a second number of second diagnostic labels, and wherein the first number is greater than the second number.

6. The system of claim 5, wherein the second diagnostic labels indicate the presence or absence of general cardiac conditions, and the first diagnostic labels indicate the presence or absence of general cardiac conditions and cardiac sub-conditions.

7. The system of any of claims 1-6, wherein the machine learning-based ECG interpretation model is a deep-learning model.

8. The system of any of claims 1-7, wherein the rules-based ECG interpretation model comprises a set of parameters based on which the ECG data is assessed to identify the presence or absence of cardiac conditions in the ECG data.

9. The system of claim 8, wherein the set of parameters of the rules-based ECG interpretation model are adapted to increase the identification of the presence of cardiac conditions in the ECG data.

10. The system of any of claims 1-9, wherein the combination unit (140) is configured to generate a same number of output diagnostic labels as the number of first diagnostic labels.

11. The system of any of claims 1-10, further comprising a display (150) configured to present the output diagnostic labels.

12. A method (200) for analyzing electrocardiogram, ECG, data of a subject, the method comprising:
processing, with a rules-based ECG interpretation model, the ECG data to generate (210) first diagnostic labels indicating presence or absence of cardiac conditions of the subject;
processing, with a machine learning-based ECG interpretation model, the ECG data to generate (220) second diagnostic labels indicating presence or absence of cardiac conditions of the subj ect;
determining (230) a feature mapping describing first diagnostic labels and second diagnostic labels associated with similar cardiac conditions; and
analyzing the first diagnostic labels based on the feature mapping and the second diagnostic labels, to generate (240) output diagnostic labels indicating presence or absence of cardiac conditions of the subject.

13. A computer program comprising computer program code means adapted, when said computer program is run on a computer, to implement the method of claim 12.
